# EUROPÄISCHE PATENTSCHRIFT

(11) **EP 3 397 120 B1**
(45) Veröffentlichungstag und Bekanntmachung des Hinweises auf die Patenterteilung: **19.01.2022**
(21) Anmeldenummer: 16822708.0
(22) Anmeldetag: 30.12.2016
(51) Int. Cl.: A47C 20/04, A47C 31/00, A61B 5/00, A47C 27/00

(54) **SCHLAF- ODER RUHEMÖBEL MIT EINER MATRATZE**
FURNITURE FOR SLEEPING OR RESTING HAVING A MATTRESS
MEUBLE DE COUCHAGE OU DE REPOS COMPORTANT UN MATELAS

(30) Priorität: 30.12.2015 DE 202015107148 U; 24.05.2016 DE 102016109524; 07.10.2016 DE 202016105633 U
(43) Veröffentlichungstag der Anmeldung: 07.11.2018
(73) Patentinhaber: DewertOkin Technology Group Co., Ltd., Jiaxing City, Zhejiang Province (CN)
(72) Erfinder: GEHRKE, Karsten, 32457 Porta Westfalica (DE); HILLE, Armin, 33659 Bielefeld (DE); LOLEY, Steffen, 49082 Osnabrück (DE); TEWS, Alexander, 33607 Bielefeld (DE)
(74) Vertreter: Kleine, Hubertus
(86) Internationale Anmeldenummer: PCT/EP2016/082912
(87) Internationale Veröffentlichungsnummer: WO 2017/114946

(56) Entgegenhaltungen:
- WO-A1-01/64103
- WO-A1-2014/185397
- CN-A- 104 257 159
- JP-A- 2004 344 675
- JP-A- 2005 253 957
- US-A1- 2008 005 838

## Beschreibung

Die Erfindung betrifft ein Schlaf- oder Ruhemöbel, insbesondere ein Bett, mit einer Matratze mit mindestens einem integrierten, in der Matratze angeordneten piezoelektrisch oder elektromagnetisch arbeitenden Sensor zur Erfassung von Vibrationen, Bewegung und/oder Schall. Das Schlaf- oder Ruhemöbel weist eine mit dem Sensor verbindbare Auswerteeinheit auf, die zur Verarbeitung und Auswertung der Signale des mindestens einen Sensors und zur Erfassung von physiologischen Parametern einer das Schlaf- oder Ruhemöbel benutzenden Person eingerichtet ist.

Im klinischen Bereich sind Überwachungsgeräte bekannt, die Atmung und/oder Herztätigkeit eines Patienten im Schlaf überwachen, um bei bedenklichen Herzfunktions- und Kreislaufparametern eingreifen zu können.

Inzwischen sind Vorrichtungen zur Überwachung des Schlafzustands anhand von physiologischen Parametern auch für nichtklinische Zwecke im Handel erhältlich. Diese Geräte, die beispielsweise auf einen Nachtisch gestellt werden, erfassen Geräusche und/oder Bewegungszustände während des Schlafs mithilfe von Mikrofonen und/oder Kameras. Aus den erfassten Informationen wird ein Schlafzustand abgeleitet, dessen zeitlicher Verlauf aufgezeichnet wird. Der aufgezeichnete Schlafverlauf kann nachträglich abgerufen und ausgewertet werden. Er kann Aufschluss darüber geben, wie tief und erholsam der Schlaf gewesen ist.

Neben Systemen, die Kamera und/oder Mikrofon einsetzen, ist ein sensorbasiertes System bekannt, bei dem ein druckempfindlicher Sensorstreifen über die Matratze gelegt wird und bei dem dieser Sensorstreifen mit einem Mobiltelefon (Smartphone) verbunden wird, das die Sensordaten aufzeichnet. Aus den Sensordaten werden unter anderem eine Herzfrequenz und eine Atemfrequenz abgeleitet. Nachteilig an den genannten nichtklinischen Systemen ist, dass die Zuverlässigkeit der Erkennung stark von der korrekten Positionierung der Überwachungsgeräte auf einen Nachtisch bzw. auf oder an der Matratze abhängt. Die Zuverlässigkeit und auch der Benutzungskomfort dieser Geräte sind dadurch eingeschränkt.

Aus der Druckschrift US 6,485,441 B2 ist ein System zur Überwachung des Schlafzustands bekannt, bei dem eine Anzahl von Vibrationssensoren in der Matratze angeordnet sind, wodurch eine Fehlpositionierung oder ein Verrutschen der Sensoren verhindert wird. Jedoch führt das Integrieren der Sensoren zu einer unerwünschten Verminderung (Dämpfung) der empfangenen Signalstärke. Gemäß der Druckschrift WO 01/64103 A1 sind in eine Matratze quer verlaufende Gräben mit v-förmigem oder rechteckigem Querschnitt eingebracht, in denen Sensoren angeordnet sind. Diese können so nah an dem Körper des Nutzers der Matratze gebracht werden. In ähnlicher Weise sind gemäß der Druckschrift JP 2005 253957 A Vertiefungen von der Oberseite ausgehend in einer Matratze ausgebildet, in denen Sensoren angeordnet sind. Die Druckschrift WO 2014/185397 A1 beschreibt eine Matratze, in der großflächige kapazitiv arbeitende Vibrationssensoren angeordnet sind, deren Signal ausgewertet wird. In ähnlicher Weise werden gemäß der Druckschrift CN 104 257 159 A Signale ausgewertet, die an Spiralfedern einer Federkernmatratze abgegriffen werden. In allen genannten Fällen werden für die Auswertung der Signale Auswerteeinrichtungen und zusätzliche Kabel sowie Stromversorgungseinheiten als Zusatzgeräte am Schlaf- oder Ruhemöbel benötigt.

Es ist eine Aufgabe der vorliegenden Erfindung, ein Schlaf- oder Ruhemöbel der eingangs genannten Art zu schaffen, bei dem der mindestens eine in die Matratze integrierte Sensor eine hohe Empfindlichkeit aufweist und Signale mit einer hohen Signalstärke bereitstellt und bei dem der Aufwand für Zusatzgeräte möglichst klein gehalten wird.

Diese Aufgabe wird durch ein Schlaf- bzw. Ruhemöbel mit den Merkmalen des unabhängigen Anspruchs gelöst. Vorteilhafte Ausgestaltungen und Weiterbildungen sind in den abhängigen Ansprüchen angegeben.

Ein erfindungsgemäßes Schlaf- oder Ruhemöbel der eingangs genannten Art weist einen elektromotorischen Möbelantrieb mit Verstellantrieben zum Verstellen von Möbelteilen auf. Weiter ist eine Steuereinrichtung zur Ansteuerung der Verstellantriebe vorgesehen. Dabei ist die Auswerteeinheit mit der Steuereinrichtung gekoppelt oder in die Steuereinrichtung integriert. Auf diese Weise können Komponenten der Steuereinrichtung, die bei dem elektromotorischen Möbelantrieb bereits vorhanden sind, auch für die Auswerteeinheit bzw. für den Sensor und/oder eine ggf. vorhandene Signaleinheit in der Matratze genutzt werden, beispielsweise eine Stromversorgungseinheit, Kommunikationseinrichtungen und/oder ein Gehäuse einschließlich der Anschlussmöglichkeiten. Zudem vereinfacht sich eine Verkabelung des Sensors, wenn die vorhandene Struktur des elektromotorischen Möbelantriebs verwendet wird.

Die Matratze weist mindestens ein Element zum Empfangen von Schall, z.B. Körperschall, Luftschall oder Bewegung, auf, mit dem der mindestens eine Sensor zur Erfassung von Vibrationen, Bewegung und/oder Schall gekoppelt ist. Der somit in die Matratze integrierte Sensor ist durch seine Positionierung vor einem Verrutschen und auch vor Beschädigung oder sonstigen Störungen geschützt, wodurch eine zuverlässige Erfassung von Vibrationen, Bewegung und Schall gegeben ist. Dazu kann das integrierte Element zum Empfangen des Schalls fest an der Matratze anbringbar ausgebildet sein. Die Matratze wirkt zudem aufgrund ihres großflächigen Kontakts mit der Person als ein guter Überträger für Körperschall. Das integrierte Element zum Empfangen von Schall, insbesondere Körperschall, nachfolgend auch Körperschallempfänger genannt, nimmt insbesondere den Körperschall auf und gibt ihn an den Sensor weiter, so dass eine gute Signalstärke gegeben ist. Bevorzugt ist der Körperschallempfänger aus einem Material gefertigt, das z.B. gegenüber den weichen in der Matratze vorkommenden Materialen, z.B. einem Schaumstoff, eine höhere Festigkeit ausweist und somit den aus der Matratze aufgenommenen Körperschall wenig dämpft und gut an den Sensor weiterleitet. Zudem weist der Körperschallempfänger bevorzugt eine Größe auf, die die des Sensors deutlich übersteigt.

Besonders bevorzugt ist der Sensor in Bereichen der Matratze angeordnet, die benachbart zu den schallerzeugenden Körperteilen der überwachten Personen liegen, also z.B. im Herz- / Lungenbereich und im Bereich des Rachens bzw. der Mundöffnung. Beispielsweise kann der Sensor an einem Rückenbereich der Matratze angeordnet sein, da derart Vibrationen, Bewegung bzw. Schall aus dem Brustraum einer die Matratze benutzenden Person besonders gut erfasst werden können, die Rückschlüsse auf Atmung und Kreislauf der Person zulassen. Eine Positionierung des Sensors in einer unteren Hälfte und insbesondere in einem unteren Drittel des Rückenbereichs ist diesbezüglich besonders geeignet. Andere Anordnungen können derart ausgestaltet sein, dass der wenigstens eine Sensor mittig in der Matratze bzw. in einem mittleren Bereich der Matratze angeordnet ist. Mit der Mitte bzw. dem mittleren Bereich ist der Volumenmittelpunkt bzw. der Schwerpunkt der Matratze gemeint. Der besondere Vorteil liegt darin, dass ein Wenden der Matratze keinen Einfluss auf die Signalqualität des Sensors hat. Dabei kann sich bevorzugt der Körperschallempfänger bis an die Auflageflächen der Matratze erstrecken und somit näher an den Schallquellen positioniert sein. Zur Produktion von Matratzen mit Sondermaßen wie Übergrößen ist dies ebenfalls von Vorteil, da die Anordnung des Sensors in der Matratze aus Fertigungssicht praktisch gleich bleibt.

Alternativ zu einer Anordnung mittig im Volumen kann auch eine Anordnung flächenmittig benachbart zu einer der Auflageflächen vorgesehen sein. Die Matratze kann dann zwar nicht mehr beidseitig eingesetzt werden, wohl aber um ihre senkreche Achse gedreht werden.

Der mindestens eine Sensor ist ein piezoelektrisch oder elektromagnetisch arbeitender Sensor. Ein solcher Sensor arbeitet robust und ist geeignet, sowohl Vibrationen (Körperschall), als auch Luftschall aufzunehmen, wobei konstruktiv festgelegt werden kann, in welchem Verhältnis diese unterschiedlichen Schallarten erfasst werden. Ein weiterer geeigneter Sensor ist ein elektromechanischer Sensor, z.B. ein mikromechanischer Beschleunigungssensor.

Weiter können auch mehrere Schall- bzw. Schwingungsaufnehmer in einem Sensor oder in verschiedenen Sensoren kombiniert werden, wobei beispielsweise ein piezoelektrisch und ein elektromagnetisch arbeitender Schall- bzw. Schwingungsaufnehmer an gleicher Position oder an verschiedenen Positionen angeordnet sind. Die verschiedenen Sensortypen zeichnen sich durch charakteristische Frequenzbereiche aus, für die sie besonders geeignet sind. Die Kombination verschiedener Sensorarten erlaubt es, ein besonders breites Frequenzspektrum analysieren zu können.

Der Sensor ist z.B. direkt oder umgeben von Schutzschichten in ein Schaummaterial der Matratze eingebettet. Beispielsweise kann der Sensor zwischen zwei Schutzschichten positioniert sein, die z.B. Moosgummi oder Filz aufweisen.

Die Sensorplatte, mit der der Sensor verbunden ist, fungiert als Element zum Empfangen von Körperschall und damit wie eine Antenne. Das Element ist bevorzugt eine Aluminium- oder Stahlplatte, die Schall möglichst ohne Dämpfung aufnimmt und weiterleitet.

Bei Anordnung des Sensors in einer Federkernmatratze kann mindestens eine der Federn des Federkerns mit einem Ende auf dem Sensor oder einer der diesen umgebenden Schutzschichten aufliegen. Diese Feder bildet in dabei den Körperschallempfänger und wirkt vorteilhaft als eine Art Antenne, die Schall bzw. Bewegung aufnimmt und auf den Sensor weiterleitet.

In vorteilhaften Ausgestaltungen des Schlaf- oder Ruhemöbels weist die Matratze einen von außen zugänglichen Steckkontakt oder mindestens zwei leitende Kontaktflächen zur Kontaktierung des Sensors auf. Auf diese Weise kann der Sensor mit Strom versorgt werden. Es kann darüber hinaus vorgesehen sein, eine Signaleinheit oder eine Auswerteeinheit sensornah in der Matratze anzuordnen. Die Signaleinheit kann z.B. einen (Vor-) Verstärker für das Sensorsignal oder auch weitere signalverarbeitende Elemente wie Filter aufweisen. Zudem kann die Signaleinheit einen Sender umfassen, um die vom Sensor erfassten Signale ggf. digitalisiert drahtlos bereitzustellen. Die Signaleinheit kann ebenfalls über den Steckkontakt bzw. die mindestens zwei leitende Kontaktflächen mit Betriebsstrom versorgt werden. Eine lokal in Sensornähe vorhandene Auswerteeinheit dient der (Vor-) Auswertung der gemessenen Sensorsignale. Die Funktionalität einer solchen oder einer externen Auswerteeinheit wird nachfolgend noch näher beschrieben. Erfindungsgemäß ist eine induktive Strom- und Signalübertragung über Induktionsspulen möglich. Erfindungsgemäß ist eine Induktionsspule im Außenbereich der Matratze, beispielsweise unterhalb eines Bezugs, angeordnet.

Die erfassten physiologischen Parameter sind beispielsweise eine Herzfrequenz, eine Atemfrequenz, ein Bewegungszustand und/oder ein Schnarchzustand der Person. Um auch kleine Signale zuverlässig auswerten zu können, weist die Auswerteeinheit vorteilhaft einen Filter, insbesondere einen Tief- oder Bandpassfilter zur Signalverarbeitung auf. Alternativ oder zusätzlich kann auch unmittelbar am Sensor bereits eine erste Signalaufbereitung erfolgen, beispielsweise indem ein Signalverstärker und/oder ein analog und/oder digital arbeitender Signalfilter benachbart zum Sensor angeordnet sind oder in ein Sensorgehäuse integriert sind. Es wird so eine weniger störanfällige Übertragung des Messsignals an die Auswerteeinheit erzielt.

Weiter kann die Auswerteeinheit einen Speicher zum Abspeichern eines Zeitverlaufs der physiologischen Parameter aufweisen. Alternativ oder zusätzlich kann die Auswerteeinheit zu diesem Zweck mit einem externen Speicher verbunden sein. Dabei kann es sich zum einen um eine Cloud handeln, also z.B. einen von einem externen Dienstleister angebotenen Speicherplatz, der dezentral und/oder verteilt von Servern bereitgestellt wird, die über das Internet erreichbar sind. Zum anderen kann es sich auch um eine sogenannte persönliche Cloud handeln, bei der ein Speicherort lokal, z. B. in Form eines NAS (Network Attached Storage) - Speichers bereitgestellt wird, der in einem Intranet erreichbar ist. Schließlich wäre auch ein unmittelbar kabelgebunden an die Auswerteinheit angebundener Massenspeicher in diesem Sinne als eine Cloud zu verstehen. Andere Formen einer kabelgebundenen, oder genauer gesagt einer verdrahtungsgebundenen Cloud umfassen USB-Massenspeichersticks oder Speicherkarten wie SD-Karten. Diese Cloud-bildenden Speicherelemente können an verschiedenen Orten und Komponenten vorgesehen sein, z.B. auch in einem PC (Personal Computer) oder einem Smartphone als Mobilgerät.

Die Auswerteeinheit kann zudem eine Überwachungseinrichtung zum Vergleichen der physiologischen Parameter mit vorgegebenen Grenzwerten aufweisen, um in einem Fall, in dem eine Gesundheitsgefahr für die Person erkannt wird, diese oder eine weitere Person gewarnt werden kann.

Zumindest zu diesem Zweck weist die Auswerteeinheit bevorzugt eine Übertragungseinheit zur Übertragung der physiologischen Parameter an ein Mobilgerät, z.B. ein Smartphone, oder eine sonstige externe Einheit auf. Die Übertragungseinheit ist bevorzugt zur drahtlosen Übertragung der physiologischen Parameter an das Mobilgerät eingerichtet, insbesondere über eine WLAN- oder Bluetooth-Übertragungsstrecke. Wenn die physiologischen Parameter an das Mobilgerät übertragen werden, kann ein Vergleich der physiologischen Parameter mit vorgegebenen Grenzwerten auch im Mobilgerät vorgenommen werden. Auch eine drahtgebundene Ankopplung an externe Einheiten ist denkbar, beispielsweise wenn die externe Einheit ein Personalruf-System in einem Pflegeheim ist.

Alternativ kann die Überwachungseinrichtung selbst extern von der Auswerteeinheit ausgebildet sein und mit der Auswerteeinheit verbunden sein. Eine solche externe Überwachungseinrichtung kann z.B. in einem Mobilgerät ausgebildet sein. Die dafür notwendige Funktionalität kann über ein entsprechendes Programm ("App") bereitgestellt werden. Die externe Überwachungseinrichtung kann auch Teil einer Alarmzentrale sein, beispielsweise in einer Pflegeeinrichtung.

In einer weiteren vorteilhaften Ausgestaltung des Schlaf- oder Ruhemöbels ist die Auswerteeinheit für die Signale des Sensors zusätzlich zur Erfassung und Auswertung von Vibrationen und Bewegung eingerichtet, die bei der Betätigung eines oder mehrerer der Verstellantriebe auftreten. Auf diese Weise kann als positiver Nebennutzen der wenigstens eine Sensor verwendet werden, um eine Fehlfunktion und/oder eine Überlastung und/oder eine Nicht-Belastung eines oder mehrerer der Verstellantriebe im Betrieb zu ermitteln. Die ermittelten Zustände deuten auf bereits eingetretene oder eventuell bevorstehende technische Probleme der Verstellantriebe hin oder auf eine Fehlnutzung.

Da der jeweilige Verstellantrieb mit den Möbelbauteilen mechanisch gekoppelt ist, ist der Sensor in der Lage, selbst die kleinsten Vibrationen und/oder Geräusche des jeweiligen Verstellantriebs erfassen zu können. Alle in diesem Zusammenhang stehenden Signale werden durch die Auswerteeinheit erfasst und mittels geeigneter Filter, beispielsweise geeigneter Bandpassfilter, als Signale der Verstellantriebe klassifiziert. Die Aussagen über den Verschleiß- und/oder Geräuschzustand des jeweiligen Verstellantriebs werden gespeichert.

Die Erfindung wird nachfolgend anhand von Ausführungsbeispielen mithilfe von Figuren näher erläutert. Es zeigen:
- Fig. 1: ein erstes Ausführungsbeispiel eines Schlafmöbels mit einer Matratze mit integriertem Sensor in einer schematischen Ansicht;
- Fig. 2: eine Wiedergabe einer zeitlichen Abhängigkeit von Sensordaten;
- Fig. 3, 4: jeweils eine schematische Schnittzeichnung einer Matratze mit einem integrierten Sensor; und
- Fig. 5 - 8: jeweils eine schematische Zeichnung zur elektrischen Kontaktierung in eines in einer Matratze angeordneten Sensors.

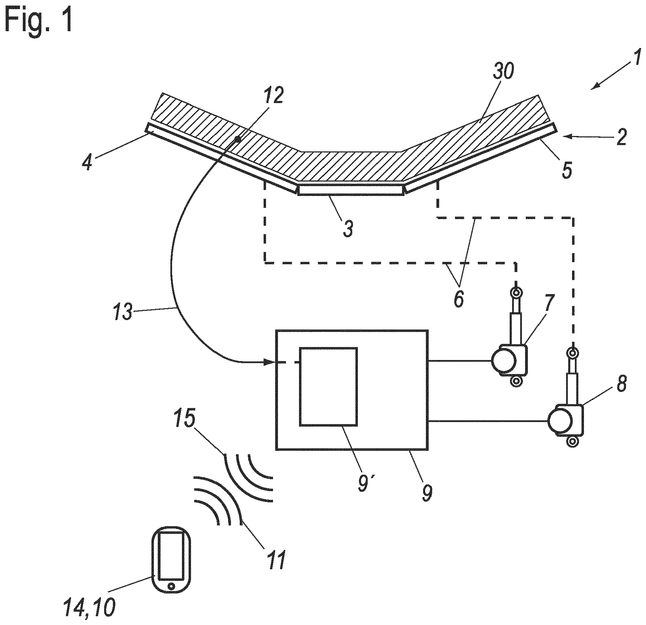

Fig. 1 zeigt ein Bett 1 als Beispiel eines Schlafmöbels mit einer Matratze 30 in einer schematischen Ansicht.

Das Bett 1 weist wenigstens ein Stützelement 2 zur Aufnahme der Matratze 30 auf. Das Bett 1 kann als ein Einzelbett für eine Person oder auch als Doppelbett für mehrere Personen ausgelegt sein. Das Stützelement 2 ist z.B. als ein Lattenrost, als ebene Stützfläche oder dergleichen ausgebildet und auf ein hier nicht dargestelltes Grundelement, z.B. ein Gestell mit Füßen, aufgelegt oder montiert.

Das Stützelement 2 weist im dargestellten Beispiel ein Rückenteil 4 und ein Beinteil 5 auf, welche relativ zu einem festen mittleren Teil 3 oder relativ zu dem Grundelement beweglich gelagert angeordnet sind. Diese bewegliche Anordnung ist beispielsweise mittels eines so genannten hier nicht dargestellten Bewegungsbeschlags realisiert. Die Bewegung ist verschiebbar und/oder schwenkbar ausgebildet.

Das in diesem Beispiel gezeigte Bett 1 ist mit einem elektromotorischen Möbelantrieb ausgestattet. Das beweglich gelagerte Rückenteil 4 und das Beinteil 5 sind dabei jeweils über eine nur schematisch gezeigte Verbindung 6 mit einem elektromotorischen Verstellantrieb 7, 8 gekoppelt. So ist das Rückenteil 4 mit dem elektromotorischen Verstellantrieb 7 gekoppelt. Zur Bewegung bzw. Verstellung des Beinteils 5 ist der elektromotorische Verstellantrieb 8 vorgesehen.

Die elektromotorischen Verstellantriebe 7, 8 sind vorliegend als Linearantriebe ausgebildet. Die Linearantriebe weisen einen oder eine Anzahl Elektromotoren auf, wobei jedem Motor ein Drehzahlreduziergetriebe mit wenigstens einer Getriebestufe nachgeschaltet ist. Dem Drehzahlreduziergetriebe kann ein weiteres Getriebe, beispielsweise in Form eines Gewindespindelgetriebes, nachgeschaltet sein, welches aus der Drehbewegung des Motors eine Linearbewegung eines Abtriebsgliedes erzeugt. Das letzte Getriebeglied oder ein damit verbundenes weiteres Glied bildet das Abtriebsglied. Das Abtriebsglied des jeweiligen elektromotorischen Verstellantriebs steht mit dem jeweiligen Möbelbauteil (Rückenteil 4, Beinteil 5) oder alternativ mit einem mit dem Grundelement verbundenes Bauteil in Verbindung, so dass bei einem Betrieb des Elektromotors des jeweiligen Verstellantriebs 7, 8 die beweglichen Möbelbauteile 4, 5 relativ zueinander bzw. relativ zum Grundelement verstellt werden.

Die elektromotorischen Verstellantriebe 7, 8 sind mit einer Steuereinrichtung 9 verbunden. Diese Verbindung kann z.B. als steckbare Kabelverbindung ausgeführt sein, was hier nicht näher dargestellt ist. Die Steuereinrichtung 9 weist eine elektrische Versorgungseinheit auf, welche die elektrische Energie, z.B. aus einem Stromversorgungsnetz, für die elektromotorischen Verstellantriebe 7, 8 bereitstellt. Dazu ist die Steuereinrichtung 9 über ein in diesem Beispiel nicht gezeigtes Netzkabel mit einem Netzstecker mit einem Netzanschluss verbindbar. Der Netzstecker leitet über das Netzkabel die eingangsseitige Netzspannung zu der elektrischen Versorgungseinheit der Steuereinrichtung 9, welche sekundärseitig eine Kleinspannung in Form einer Gleichspannung abgibt.

Alternativ hierzu ist der Steuereinrichtung 9 eine externe netzabhängige Spannungsversorgung mit Netzeingang und mit sekundärseitigem Kleinspannungsausgang vorgeschaltet, welche über die Leitung die Kleinspannung in Form einer Gleichspannung zuführt.

In einer alternativen Ausgestaltung ist die Steuereinrichtung nicht in einem separaten Gehäuse angeordnet, sondern in einen der Verstellantriebe 7, 8 integriert. Dieser Verstellantrieb stellt dann einen Hauptantrieb dar, an den ggf. weitere Verstellantriebe angeschlossen werden können.

In einer weiteren alternativen Ausgestaltung eines elektromotorischen Möbelantriebs kann die Steuereinrichtung verteilt im System angeordnet sein, derart, dass jeder der Verstellantriebe 7, 8 selbst über eine Motorsteuerung verfügt und eine Bus-Kommunikationsschnittstelle aufweist, über die die Verstellantriebe 7, 8 untereinander und mit weiteren Komponenten verbunden sind. Dabei kann vorgesehen sein, dass wenigsten einer der Verstellantriebe 7, 8 ein eigenes Netzteil zu seiner Stromversorgung oder zur Versorgung mehrerer oder aller vorhandenen Verstellantriebe und/oder ggf. weiterer Systemkomponenten aufweist.

Es ist eine Handbedienung 10 vorgesehen, die Bedienelemente aufweist, mit denen die elektromechanischen Verstellantriebe 7, 8 über die Steuereinrichtung 9 steuerbar sind. Die Handbedienung 10 kann in einem Ausführungsbeispiel über ein Kabel mit der Steuereinrichtung 9 verbunden sein. Alternativ kann die Handbedienung 10 mit einer Übertragungseinrichtung für eine drahtlose Übertragung von Signalen zur Steuereinrichtung 9 versehen sein. Die drahtlose Übertragung kann durch eine Funkübertragungsstrecke, eine optische Übertragungsstrecke (z.B. für Infrarotlicht) und/oder eine Ultraschallübertragungsstrecke realisiert sein, wobei die Steuereinrichtung 9 mit einer jeweiligen entsprechenden Empfangseinheit ausgerüstet ist. Weiter alternativ kann die Handbedienung auch die Steuereinrichtung für die Verstellantriebe bilden, z.B. indem der Betriebsstrom der Verstellantriebe direkt über Schalter der Handbedienung geschaltet wird.

Bei dem dargestellten Ausführungsbeispiel übernimmt ein Mobilgerät 14 die Funktion der Handbedienung 10. Das Mobilgerät 14 kann insbesondere ein handelsübliches Mobiltelefon ("Smartphone") oder ein Tablet-Computer sein. Bevorzugt ist eine Software ("App") für die Funktion als Handbedienung 10 auf dem Mobilgerät 14 installiert. Steueranweisungen an die Verstellantriebe 7, 8 können so über eine drahtlose Übertragungsstrecke 11 von dem als Handbedienung genutzten Mobilgerät 14 an die Steuereinrichtung 9 gesendet werden.

Die drahtlose Übertragungsstrecke 11 kann beispielsweise auf einem WLAN (Wireless Lokal Area Network) oder Bluetooth Übertragungsweg basieren.

Anmeldungsgemäß ist bei dem dargestellten Bett 1 ein Sensor 12 vorgesehen, der Vibrationen, Bewegung und/oder Schall detektiert und in die Matratze 30 integriert. In alternativen Ausgestaltungen der Matratze 30 können mehrere Sensoren 12 vorgesehen sein.

Der Sensor 12 ist beispielsweise als piezoelektrisches Bauteil oder als elektromagnetisches oder elektromechanisches Bauteil ausgebildet und ist empfindlich für Schwingungen bzw. Bewegungen der Unterlage, an oder auf der er befestigt ist. Derartige Vibrationen umfassen insbesondere auch Körperschall. Unter "Bewegungen" sind insbesondere niederfrequente Schwingungen und Auslenkungen des Sensors 12 zu verstehen, deren Frequenz im Hertz- oder Sub-Hertz-Bereich liegt. Zusätzlich kann der Sensor 12 empfindlich für (Luft-) Schallwellen sein und in diesem Sinne als ein Mikrofon fungieren.

Der Sensor 12 ist über ein Sensorkabel 13 mit der Steuereinrichtung 9 verbunden. Falls benötigt, wird über das Sensorkabel 13 eine Stromversorgung für den Sensor 12 bereitgestellt und es werden vom Sensor 12 ausgegebene Signale an die Steuereinrichtung 9 weitergeleitet. In einer alternativen Ausgestaltung kann der Sensor 12 über eine drahtlose Verbindung, beispielsweise eine Funkverbindung, mit der Steuereinrichtung 9 gekoppelt sein. In dem Fall ist der Sensor 12 mit einer eigenen Energieversorgung, beispielsweise in Form einer gegebenenfalls wiederaufladbaren Batterie versehen.

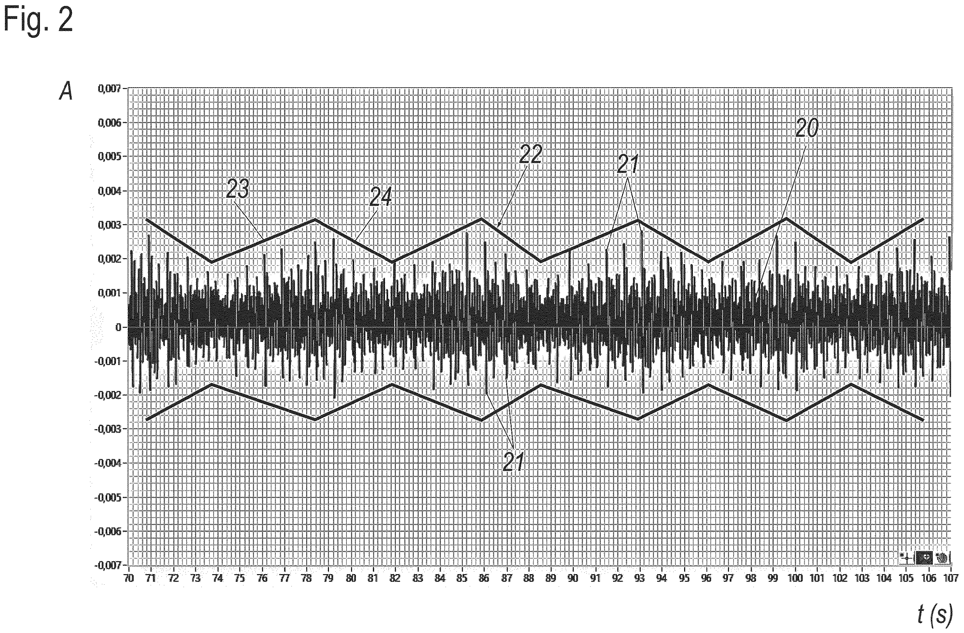

Die Steuereinrichtung 9 umfasst eine Auswerteeinheit 9' zur Verarbeitung und Auswertung der vom Sensor 12 gelieferten Signale. Die Auswerteeinheit 9' umfasst z.B. Verstärker und Filtereinheiten, die es ermöglichen, aus dem vom Sensor 12 übermittelten Signal auf bestimmte Körperfunktionen einer sich im Bett 1 befindenden Person zu schließen. Insbesondere ist die Auswerteeinheit dazu eingerichtet, aus den Signalen des Sensors 12 physiologische Parameter der Person zu ermitteln. Solche Parameter betreffen beispielsweise Herz- und Kreislauffunktionen und umfassen z.B. eine Herzfrequenz und eine Atemfrequenz. Weiter kann ermittelt werden, ob die sich im Bett befindende Person schnarcht. Zudem werden Bewegungen der Person erfasst. Details zur Ermittlung der genannten Parameter aus den Signalen des Sensors 12 werden weiter unten im Zusammenhang mit Fig. 2 näher erläutert.

Die ermittelten Parameter werden entweder unmittelbar oder nach Zwischenspeicherung in der Auswerteeinheit 9' als drahtlose Signale 15 an das Mobilgerät 14 übertragen, das mit einer entsprechenden Software ("App") ausgestattet ist, die eine Auswertung und bevorzugt grafische Darstellung der Zeitabhängigkeit der ermittelten Schlafparameter ermöglicht. Als Übertragungsweg 15 für Daten der Auswerteeinheit 9' kann beispielsweise wiederum WLAN (Wireless Lokal Area Network) oder Bluetooth eingesetzt werden.

Zudem kann in der Auswerteeinheit 9' bzw. der Steuereinrichtung 9 ein Vergleich der gemessenen physiologischen Parameter mit vorgegebenen Grenzwerten für diese Parameter vorgesehen sein. Werden die ermittelten Parameter unmittelbar, das heißt ohne längere Zwischenspeicherung in der Auswerteeinheit 9', während der Schlafphase an das Mobilgerät 14 übertragen, kann alternativ oder zusätzlich dort ein solcher Vergleich erfolgen. Bei Über- oder Unterschreiten der Grenzwerte bzw. bei einem Verlassen eines oder mehrerer der Parameter aus einem vorgegebenen Bereich ist vorgesehen, dass die Auswerteeinheit 9' bzw. die Steuereinrichtung 9 oder das Mobilgerät 14 ein Alarmsignal ausgibt. Dieses Alarmsignal kann optisch und/oder akustisch unmittelbar von der Auswerteeinheit 9' bzw. der Steuereinrichtung 9 oder dem Mobilgerät 14 ausgegeben werden. Alternativ oder zusätzlich kann vorgesehen sein, dass das Mobilgerät 14 eine Alarmmeldung über eine weitere, hier nicht dargestellte drahtlose Übertragungsstrecke (z.B. WLAN, Mobilfunknetz) abgibt. Auf diese Weise kann eine weitere Person benachrichtigt werden, falls sich ungewöhnliche Schlafparameter zeigen. Das dargestellte Bett 1 bzw. der elektromotorische Möbelantrieb mit dem Sensor 12 kann so auch zur klinischen Überwachung bzw. zur Personenüberwachung oder zur Überwachung von Kleinkindern zum Schutz vor plötzlichem Kindstod eingesetzt werden. Beispielsweise kann eine Alarmmeldung abgegeben werden, wenn eine Person das Bett verlassen hat, ggf. wenn eine Person das Bett seit einer vorbestimmten Zeit verlassen hat oder wenn keine oder nur als kritisch angesehene physiologische Parameter detektiert werden.

Vorteilhaft ist die Verbindung zwischen dem Sensor 12 und der Auswerteeinheit 9' innerhalb des Betts 1 angeordnet, wodurch verhindert ist, dass das Sensorkabel 13 außerhalb des Bettes 1 verlegt werden muss. Die Integration des Sensors 12 in der Matratze stellt eine jederzeit korrekte Positionierung des Sensors 12 und damit eine zuverlässige Auswertung der Daten des Sensors 12 sicher. Es kann vorgesehen sein, den Sensor 12 mit der Auswerteeinheit 9' in einer Baueinheit zusammenzufassen.

Bei dem Ausführungsbeispielen der Fig. 1 ist die Auswerteeinheit 9' für die Signale des Sensors 12 in die Steuereinrichtung 9 integriert. Alternativ ist es möglich, die Auswerteeinheit 9' separat von der Steuereinrichtung 9 in einem eigenen Gehäuse auszubilden. Zur Übertragung der ermittelten physiologischen Parameter kann die Auswerteeinheit 9' dann mit der Steuereinrichtung 9 elektrisch gekoppelt sein. Auch ist eine Nutzung einer solchen autarken Auswerteeinheit 9' losgelöst von der Steuereinrichtung 9 möglich, insbesondere wenn im Gehäuse der Auswerteeinrichtung 9' bereits eine Übertragungseinheit zur drahtlosen Übertragung der ermittelten physiologischen Parameter und/oder vorverarbeiteter Signale des Sensors 12 an das Mobilgerät 14 oder eine andere externe Komponente vorhanden ist.

Fig. 2 zeigt einen Ausschnitt eines gemessenen Signals 20 des Sensors 12 in einem Diagramm. Auf der horizontalen Achse ist der Zeitverlauf t in Sekunden angegeben. Auf der vertikalen Achse ist eine Signalamplitude A in willkürlichen Einheiten dargestellt.

Der gezeigte Ausschnitt des Signalverlaufs des Signals 20 ist während einer ruhigen Schlafphase ohne Bewegung und ohne Schnarchen der beobachteten Person. Eine Bewegung der Person äußert sich in Amplituden, die die dargestellte um einen Faktor von einigen 10-100 übersteigen. Bewegungen lassen sich daher sehr leicht identifizieren. Auch ein Schnarchen und die damit einhergehenden Vibrationen sind von dem dargestellten Signalverlauf deutlich unterscheidbar, da sie sich in einer um ein mehrfaches größeren Amplitude widerspiegeln.

In dem in Fig. 2 gezeigten Verlauf des Signals 20 sind regelmäßige Peaks 21 beobachtbar, die vom Herzschlag der Person herrühren und nachfolgend Herzschlag-Peaks 21 genannt werden. Aus dem Abstand der Herzschlag-Peaks 21 kann eine Herzfrequenz ermittelt werden. Der zeitliche Abstand benachbarter Herzschlag-Peaks 21 lässt Aussagen über die Puls-Gleichmäßigkeit zu, die ein Maß für die Tiefe des Schlafes sein kann.

Weiterhin ist in Fig. 2 zu erkennen, dass die Amplitude der Herzschlag-Peaks 21 regelmäßig mit einer geringeren Frequenz variiert. Diese Variation wird durch eine Einhüllende 22 wiedergegeben. Die Einhüllende 22 zeigt sich abwechselnde ansteigende Flanken 23 und abfallende Flanken 24. Der Verlauf der Einhüllenden 22 ist mit der Atmung der Person korreliert. Die ansteigenden Flanken 23 kennzeichnen eine Einatmungsphase und die absteigende Flanke 24 eine Ausatemphase.

Das Beispiel der Fig. 2 zeigt, wie aus den Signalen des Sensors 12 auf Herz-Kreislaufparameter geschlossen werden kann, vorliegend auf Puls- und Atmung. Auf ähnliche Weise können weitere Schlafparameter, wie Bewegungszustände und ein Schnarchen ermittelt werden.

Zur Auswertung der Signale 20 erfolgt eine Filterung der Rohsignale des Sensors 12, insbesondere mittels eines Tiefpassfilters. Auch die Verwendung eines Bandpassfilters mit geeigneten Eckfrequenzen ist möglich. Tiefpass- bzw. Bandpassfilter dienen der Eliminierung von Störfrequenzen. Bevorzugt erfolgt die Verarbeitung der Signale mit Hilfe eines digitalen Signalprozessors (DSP).

Der Sensor 12 kann zusätzlich oder alternativ auch einer Überwachung der korrekten Funktion des elektromotorischen Antriebs dienen. Eine Betätigung der Verstellantriebe 7, 8 führt zu einer Bewegung der bewegten Möbelteile, beispielsweise des Rückenteils 4 und/oder des Beinteils 5. Zusätzlich resultiert die Betätigung der Verstellantriebe 7, 8 in Vibrationen dieser Möbelteile und auch des gesamten Möbels, die ebenfalls vom Sensor 12 erfasst werden. Diese Vibrationen treten in einem typischen Frequenzbereich auf. Der Signalverlauf spiegelt die Motorbewegung der Verstellantriebe 7, 8 wider. Ein erster typischer relevanter Frequenzbereich liegt im Bereich der Motordrehzahl der Motoren der Verstellantriebe 7, 8. In diesem Frequenzbereich zeigen sich Fehler am Motor selbst oder einem Abtriebszahnrad. Ein weiterer typischer relevanter Frequenzbereich entspricht einem ganzzahligen Bruchteil gemäß einem Übersetzungsverhältnis des Getriebes, das etwa 1:30 bis 1:50 beträgt. In diesem Frequenzbereich deuten sich Fehler in nachgeordneten Getriebestufen oder Wälzlagern an. Ein dritter typischer Frequenzbereich liegt im Bereich von Quietschgeräuschen von Scharnieren, die Teil eines Möbelbeschlags sind. Form und Amplitude sind zum einen typisch für den verwendeten Verstellantrieb 7, 8, zum anderen geben sie Aufschluss über die korrekte Funktion der Verstellantriebe 7, 8 sowie deren Verschleißzustand.

Auch eine Überbelastung einer der Verstellantriebe 7, 8 kann anhand der Signalform der Signale des Sensors 12 erkannt werden. Der Sensor 12 kann somit beispielsweise als Einklemmschutz fungieren, wobei die Steuereinrichtung 9 bei Überbelastung eines der Verstellantriebe 7, 8 diesen Antrieb stoppt bzw. in Gegenrichtung laufen lässt. Auch eine Unterlast am Verstellantrieb 7, 8 kann ein Indiz für ein Einklemmen sein, beispielsweise wenn ein Möbelteil (Rückenteil 4, Beinteil 5) abgelassen wird und der Verstellantrieb 7, 8 nahezu kraftlos betrieben wird, deutet dieses auf ein Einklemmen eines Körperteils unter dem sich hinab senkenden bewegten Möbelteil hin. Ein belastungslos betriebener Verstellantrieb 7, 8 ist ebenfalls anhand der Signale des Sensors 12 identifizierbar.

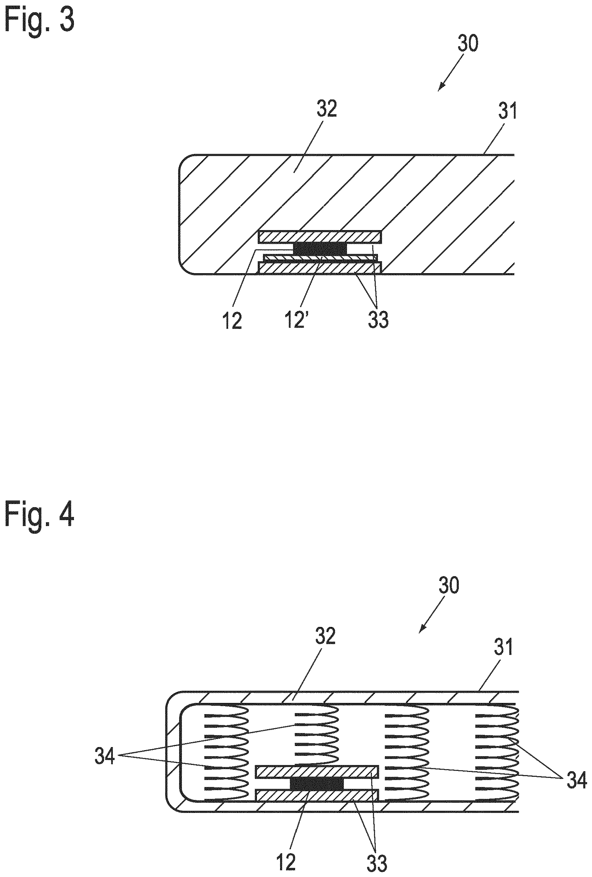

Im Zusammenhang mit den Figuren 3 und 4 ist eine mögliche Anordnung des Sensors 12 in der Matratze 30 dargestellt.

Fig. 3 zeigt einen Querschnitt durch einen Teilbereich der Matratze 30 in einer ersten Ausgestaltung. Die Matratze 30 weist einen Bezug 31 auf, der einen Kern aus einem Schaummaterial 32 umgibt. Als Schaummaterial 32 ist beispielsweise ein Kaltschaum gebräuchlich.

Der Sensor 12 ist im dargestellten Ausführungsbeispiel in einem unteren Bereich der Matratze 30 in eine Aussparung eingesetzt, die in den Kern aus Schaummaterial 32 eingebracht ist. Der Sensor 12 ist auf einer Sensorplatte 12' angeordnet und fest mit dieser verbunden, z.B. durch eine Klebeverbindung. Die Sensorplatte 12' dient hierbei als Empfangselement (Antenne) und nimmt Körperschall großflächig auf und leitet ihn an den Sensor 12 weiter. Die Sensorplatte 12' kann z.B. eine Aluminium oder Stahlplatte sein, ist also aus einem harten Material gefertigt, das aufgenommenen und weitergeleiteten Schall möglichst wenig dämpft. Alternativ kann auch ein möglichst harter Kunststoff als Material für die Sensorplatte 12' verwendet werden.

Um einen Schutz des Sensors 12 zu gewähren und um eine gute akustische Anbindung des Sensors 12 bzw. der Sensorplatte 12' an das Schaummaterial 32 zu erzielen, ist die Anordnung aus Sensor 12 und Sensorplatte 12' zwischen zwei Schutzlagen 33 aus einem ebenfalls nachgiebigem Material eingebettet, das jedoch fester ist als das Schaummaterial. Das Material der Schutzlagen 33 kann beispielsweise aus Moosgummi oder Filz sein.

Von den gezeigten Sensoranordnungen können bevorzugt mehrere in der Matratze 30 angeordnet sein. Auch mit relativ kleinen Sensorplatten 12' wird dann zusammengenommen eine ausreichend große aufsummierte Signalstärke erzielt. Kleinere Sensorplatten 12' bzw. Sensoranordnungen haben gegenüber einer größeren den Vorteil, dass sie den Schlafkomfort des Benutzers nicht beeinträchtigen. Eine geeignete Größe der Sensorplatten 12' liegt bei etwa 30 bis 50 Millimetern Durchmesser oder Kantenlänge.

Anstelle einer Anordnung benachbart zu einer der Auflageflächen der Matratze 30 kann auch eine mittige Anordnung zwischen Ober- bzw. Unterseite der Matratze 30 vorgesehen sein, beispielsweise indem von einer der Seiten ein Einschnitt in das Schaummaterial 32 vorgenommen wird, wodurch eine Tasche gebildet ist, in die das Sandwich aus Sensor 12 und Schutzschichten 33 eingeschoben wird. Der Einschnitt kann von einer Längsseite der Matratze 30 aus erfolgen. Soll ein Sensor 12 dichter im Kopfbereich oder im Brustbereich einer ruhenden Person angeordnet sein, kann als Alternative ein Einschnitt in die Matratze 30 von der Querseite aus erfolgen.

Fig. 4 zeigt die Anordnung des Sensors 12 in einer als Federkernmatratze ausgebildeten Matratze 30. Diese weist wiederum einen Bezug 31 auf, der eine äußere Schicht eines Schaummaterials 32 umgibt. Im Inneren ist mit Hilfe von Federn 34 ein Federkern gebildet. Beim dargestellten Ausführungsbeispiel ist der Sensor 12 im Bereich des Federkerns angeordnet, wobei eine der Federn 34 auf dem Sandwich aus Sensor 12 und Schutzschichten 33 endet. Auf diese Weise wirkt die entsprechende Feder 34 als ein Körperschallempfänger, d.h. wie eine Antenne, die akustische Schwingungen bzw. Bewegungen aufnimmt, ggf. resonant verstärkt und auf den Sensor 12 weiterleitet.

In alternativen Ausgestaltungen ist es denkbar, die Anordnung aus Sensor 12 und Schutzschichten 33 in einen mittleren Bereich der Matratze 30 so anzuordnen, dass eine Feder 34 von oben und von unten auf den Sensor 12 wirkt. Auch eine Anordnung innerhalb der Schicht aus dem Schaummaterial 32 analog zu dem in Fig. 3 gezeigten Beispiel ist möglich.

In einer weiteren alternativen Ausgestaltung kann der Sensorinnen in einem gegebenenfalls abnehmbaren Bezug der Matratze angeordnet sein. Um den Bezug dennoch waschen zu können kann der Sensor abtrennbar befestigt sein, z. B. mit Druckknöpfen, einem Klettverschluss, einem Reißverschluss. Auch kann eine Aufnahme und Positionierung des Sensors in einer z.B. angenähten Tasche erfolgen.

Dabei kann ein Körperschallempfänger z.B. in Form von Kunststofffolien oder Kunststoffelemente am Bezug angeordnet sein, mit dem der Sensor verbunden ist oder mit den zuvor genannten Befestigungsmitteln verbunden werden kann.

Alternativ kann der Sensor auch fest mit dem Körperschallempfänger gekoppelt sein und dieser über die genannten Befestigungsmittel lösbar an dem Bezug befestigt werden.

Bei allen bislang beschriebenen Beispielen ist der Sensor eine zwar mit dem Körperschallempfänger gekoppelte, aber ansonsten von diesem separate Einheit. Alternativ dazu kann in allen gezeigten oder beschriebenen Fällen, sowohl bei Anordnung im Kern der Matratze, als auch bei Anordnung im Bezug, der Sensor integriert mit dem Körperschallempfänger ausgebildet sein. Dazu kann als Sensor auf Basis eines polarisierten Kunststoffes, z.B. auf Fluor-Basis, aufgebaut sein, der piezoelektrische Eigenschaften aufweist. Der Sensor selbst kann dann flächig, ggf. netzförmig, ausgebildet sein und in dem Sinne die Funktionen eines flächigen Körperschallempfängers und eines signalabgebenden Sensors vereinen.

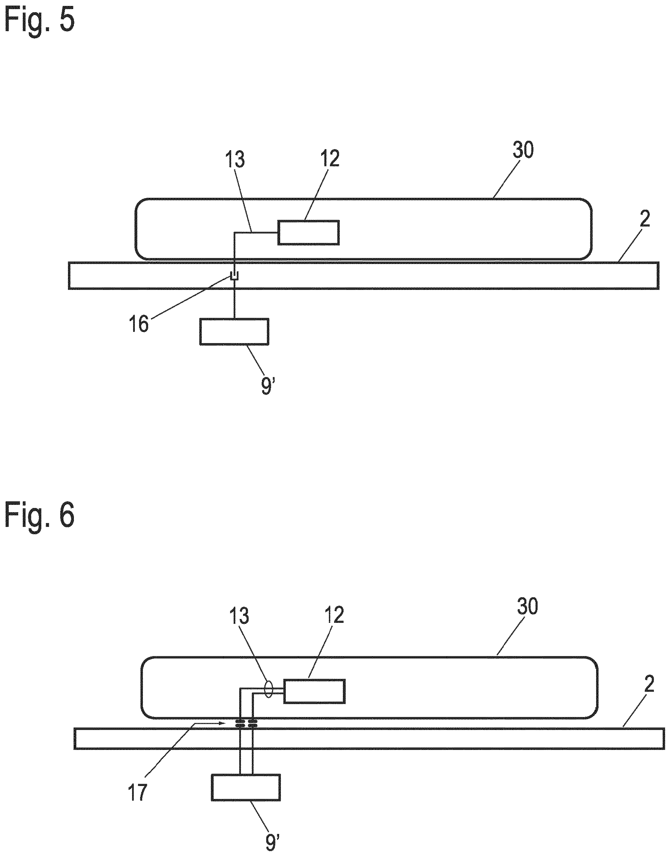

Im Zusammenhang mit den Figuren 5 bis 8 ist die elektrische Kontaktierung eines in einer Matratze 30 angeordneten Sensors 12 dargestellt. Die in diesen Figuren gezeigte elektrische Kontaktierung kann beispielsweise im Zusammenhang mit den Sensoren 12 in den Ausführungsbeispielen der Figuren 3 und 4 eingesetzt werden. Auch wenn jeweils in den Figuren nur der Sensor 12 dargestellt ist, versteht es sich, dass dieser wie bei den Ausführungsbeispielen der Fig. 3 und 4 mit einem Element zum Empfangen von Körperschall, z.B. der Sensorplatte 12' oder der Feder 34, gekoppelt ist.

Von dem Bett (vgl. Bezugszeichen 1 in den Fig. 1) ist lediglich das Stützelement 2 dargestellt, auf das die Matratze 30 aufgelegt ist. Das Stützelement 2 kann beispielsweise ein Lattenrost oder, bei sogenannten Boxspringbetten, eine plattenförmige Auflagefläche sein. Anstelle einer Steuerrichtung (vgl. Bezugszeichen 9 in den Fig. 1), die Teil eines elektromotorischen Möbelantriebs ist, ist vorliegend die Auswerteeinheit 9' zur Auswertung der Sensorsignale des Sensors 12 als separate Einheit ausgeführt. Es versteht sich, dass die Auswerteeinheit 9' alternativ auch in eine hier nicht dargestellte Steuereinheit integriert sein kann.

in Fig. 5 eines nicht erfindungsgemäßen Beispiels ist zunächst eine elektrische Kontaktierung des Sensors 12 über ein Sensorkabel 13 und einen Steckkontakt 16 gezeigt. Der Steckkontakt 16 ermöglicht es, den Sensor 12 von der Auswerteeinheit 9' abzukoppeln, beispielsweise um die Matratze 30 zu lüften oder zu tauschen. Der Steckkontakt 16 ist symbolisch einpolig dargestellt. Es versteht sich, dass in einer Umsetzung der gezeigten Anordnung ein mehrpoliger Steckkontakt 16 eingesetzt werden kann, der sowohl ggf. eine Stromversorgung für den Sensor 12 bereitstellt, als auch Signale des Sensors 12 zur Auswerteeinheit 9' überträgt. Der Steckkontakt 16 kann unmittelbar benachbart zur Matratze 30 positioniert sein oder auch über einen Kabelabschnitt einer bestimmten Länge, die eine flexiblere Kontaktierung am Stützelement 2 oder einer anderen Bettkomponente oder an der Auswerteeinheit 9' ermöglicht.

Eine Weiterbildung der Anordnung aus Fig. 5 ist in dem nicht erfindungsgemäßen Beispiel der Fig. 6 gezeigt. Bei prinzipiell gleichem Aufbau ist der Steckkontakt 16 durch eine Anordnung von Kontaktflächen 17 ersetzt. Die Kontaktflächen 17 ermöglichen eine Kontaktierung zwischen der Matratze 30 und der Auswerteeinheit 9', ohne dass manuell ein Stecker eingesteckt werden müsste. Die Kontaktflächen 17 sind am Stützelement 2 bzw. der Matratze 30 jeweils so positioniert, dass beim Auflegen der Matratze 30 auf das Stützelement 2 automatisch eine zumindest zweipolige Kontaktierung stattfindet. Wiederum können separate Kontakte für eine Stromversorgung und für Sensorsignale vorgesehen sein. Die Kontaktflächen 17 können unmittelbar am Stützelement 2 angeordnet sein, oder mittels eines Trägerelements, das seinerseits am Stützelement 2 montiert wird. Letzteres bietet eine einfache Nachrüstmöglichkeit.

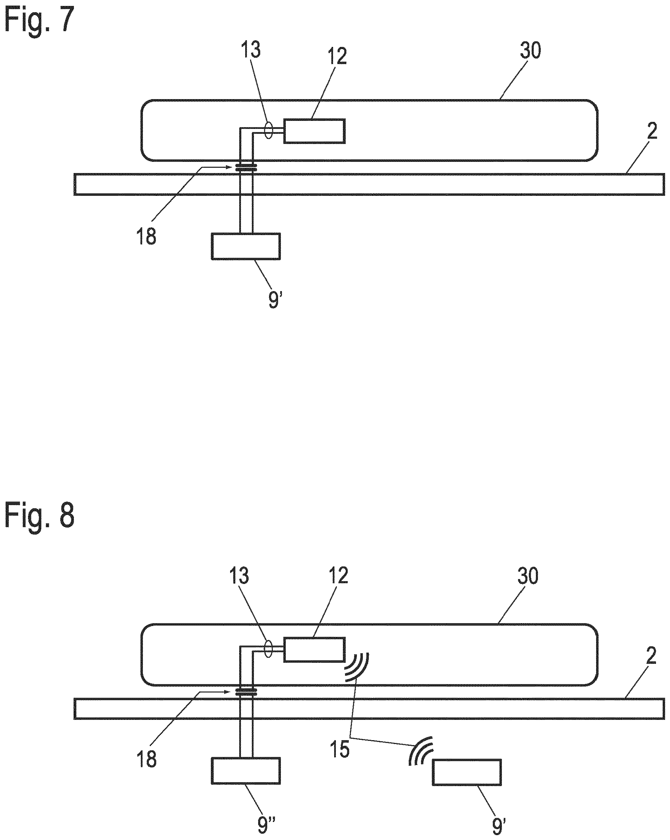

Es ist auch denkbar, über nur zwei Kontakte sowohl den Sensor mit Strom zu versorgen, als auch moduliert auf die Stromversorgung die Signale des Sensors 12 an die Auswerteeinheit 9' zu übertragen. Dieses ist für das in Fig. 7 gezeigte Ausführungsbeispiel vorteilhaft, da die Zahl der sinnvoll in die Matratze bzw. die Stützeinheit integrierbaren Kontakte kleiner ist als bei Verwendung des Steckkontakts 16 gemäß Fig. 5 eines nicht erfindungsgemäßen Beispiels.

Der Grund ist, dass zum Ausgleich von Toleranzen in der Positionierung der Matratze 30 die Kontaktflächen 17 eine gewisse Mindestgröße und einen gewissen Mindestabstand haben sollten. Als Material der Kontaktflächen ist bevorzugt ein flexibles Material, insbesondere ein leitendes Textilmaterial einsetzbar. Beispielsweise kann in bestimmten Bereichen in der Matratze 30 ein leitfähiges Textil, beispielsweise ein von einem Kupferfaden durchwirktes Textil eingesetzt werden. Die am Stützelement 3 ausgebildeten (Gegen-) Kontaktflächen 17 können dann aus einer harten leitenden Fläche gebildet sein.

Eine erfindungsgemäße Anordnung zur Ubertragung von Energie und Sensorsignalen ist in Fig. 7 dargestellt. Bei diesem Ausführungsbeispiel werden die elektrischen Signale induktiv durch Induktionsspulen 18 übertragen. Dazu wird die primärseitige, mit der Auswerteeinheit 9' verbundene Induktionsspule 18 mit Wechselspannung beaufschlagt. Die in der sekundärseitigen Induktionsspule 18 induzierte Spannung wird dem Sensor 12 zur Stromversorgung zugeführt. Auch bei dieser Anordnung kann vom Sensor 12 das Sensorsignal als ein hochfrequentes Signal auf den zu seiner Versorgung fließenden Strom aufmoduliert werden, welcher wiederum nach ebenfalls induktiver Übertragung durch die Induktionsspulen 18 auf die Auswerteeinheit 9' übertragen wird. Eine derartige Modulation ist auch als Power-Line-Modulation bekannt.

Fig. 8 zeigt eine Abwandlung der Anordnung gemäß Fig. 7. Beim Ausführungsbeispiel der Fig. 8 ist zur Energieversorgung des Sensors 12 ebenfalls eine Anordnung von Induktionsspulen 18 vorgesehen, die in diesem Ausführungsbeispiel primärseitig mit einer von der Auswerteeinheit 9' getrennten Stromversorgungseinheit 9" gekoppelt ist.

Eine Übertragung der Signale des Sensors 12 zur Auswerteeinheit 9' erfolgt in Form von drahtlosen Signalen 15 über eine Funkstrecke. Diese kann beispielsweise gemäß der Bluetooth- oder ZigBee-Spezifikation ausgebildet sein. Es versteht sich, dass eine Aufteilung in eine induktive Stromversorgung des Sensors 12 und eine Übertagung der Sensorsignale als drahtlose Signale 15 auch möglich ist, wenn keine separate Stromversorgungseinheit 9" vorhanden ist, sondern die Stromversorgungseinheit 9" in die Auswerteinheit 9' integriert ist. Auch Funkstrecken mit proprietären Übertragungsprotokollen sind denkbar.

Ferner sind zuvor genannte Merkmale miteinander kombinierbar. Somit ist gemäß einer nicht näher dargestellten Alternative eine Matratze 30 mit einem damit verbundenen Sensor 12 und mit einer damit verbundenen Auswerteeinheit 9' denkbar. Die Matratze 30 umfasst somit den wenigstens einen Sensor 12 und die Auswerteeinheit 9'. Dabei kann die Auswerteeinheit 9' in der Nähe eines Reißverschlusses des Bezuges 31 vorgesehen sein. Die Auswerteeinheit 9' kann dabei im Randbereich bzw. im Oberflächenbereich der Matratze 30 angeordnet sein.

In einer Ausführung ist die Auswerteeinheit 9' dabei zur Aufnahme wenigstens eines elektrischen Energiespeichers ausgebildet. Es kann z.B. ein Batteriefach vorgesehen sein. Bevorzugt weist die Auswerteeinheit 9' einen wiederaufladbaren elektrischen Energiespeicher auf, der insbesondere geeignet ist, die Auswerteeinheit 9' für einen Zeitraum von wenigstens 24 Stunden zu versorgen. Das Aufladen des Energiespeichers erfolgt in einer Ausführung gemäß der Beschreibung zu den Figuren 7 oder 8, wobei in der Matratze 30 oder der Auswerteeinheit 9' die dort genannte sekundäre Induktionsspule 18 angeordnet ist. Durch die zuvor beschriebene Ausgestaltung ist es nunmehr möglich, der Auswerteeinheit 9' eine zeitweise Übertragung von elektrischer Energie durch die Induktionsspulen 18 zur Verfügung zu stellen, welche dann durch den elektrischen Energiespeicher zwischengespeichert wird und zum Betrieb der Auswerteeinheit 9' zur Verfügung steht.

In einer Ausgestaltung erfasst die Auswerteeinheit 9' die Anwesenheit einer auf der Matratze 30 ruhenden Person und deaktiviert das Übertragen von Energie durch die Induktionsspulen 18. Auf diese Weise wird Energie eingespart und eine Abgabe von elektromagnetischen Verunreinigungen auf ein Minimum reduziert. In einer weiteren Ausgestaltung erfasst die Auswerteeinheit 9' der Matratze nicht nur die Signale 20 des wenigstens eines Sensors 12, sondern vollzieht auch die Auswertung der Sensorsignale, z.B. zur Ermittlung der Schlafphasen und zur Analyse der jeweiligen Schlafphase und fasst nach dem Ende der letzten Schlafphase alle Messdaten zu einem Datensatz zur drahtlosen Übertragung an ein Mobilgerät 14 oder zur Übertragung in ein Datennetzwerk zusammen. Dazu weist die Auswerteeinheit 9' bevorzugt einen Speicher auf, der die ermittelten Parameter bis zur Übertragung an das Mobilgerät 14 zwischenspeichern kann. Um keine Daten zu verlieren, auch wenn eine Übertragung für einen längeren Zeitraum nicht stattfindet, sollte der Speicher eine Kapazität haben, die groß genug ist um Daten mehrerer Schlafphasen aufzunehmen, z.B. Daten einer Woche. Der Speicher kann z.B. ein nicht-flüchtiger, wiederbeschreibbarer Speicher sein, z.B. ein Flash-Speicher.

### Bezugszeichenliste

- 1: Bett
- 2: Stützelement
- 3: mittlerer Teil des Stützelements
- 4: Rückenteil
- 5: Beinteil
- 6: Verbindung
- 7, 8: Verstellantrieb
- 9: Steuereinrichtung
- 9': Auswerteeinheit
- 9": Stromversorgungseinheit
- 10: Handbedienung
- 11: Übertragungsstrecke
- 12: Sensor
- 12': Sensorplatte
- 13: Sensorkabel
- 14: Mobilgerät
- 15: drahtlose Signale
- 16: Steckkontakt
- 17: Kontaktflächen
- 18: Induktionsspulen

- 20: Signal
- 21: Herzschlag-Peak
- 22: Einhüllende
- 23: ansteigende Flanke (Einatmung)
- 24: abfallende Flanke (Ausatmung)

- 30: Matratze
- 31: Bezug
- 32: Schaummaterial
- 33: Schutzlage
- 34: Feder

## Patentansprüche

1. Schlaf- oder Ruhemöbel, insbesondere Bett (1), aufweisend
- eine Matratze (30) mit mindestens einem in einem Schaummaterial (32) der Matratze (30) eingebetteten piezoelektrisch oder elektromagnetisch arbeitender Sensor (12) zur Erfassung von Vibrationen, Bewegung und/oder Schall, wobei die Matratze (30) mindestens eine Sensorplatte (12') aufweist, die als Element zum Empfangen von Körperschall fungiert und mit der der mindestens eine Sensor (12) gekoppelt ist,
- weiter aufweisend eine mit dem Sensor (12) verbundene Auswerteeinheit (9') die zur Verarbeitung und Auswertung der Signale des mindestens einen Sensors (12) und zur Erfassung von physiologischen Parametern einer das Schlaf- oder Ruhemöbel benutzenden Person eingerichtet ist,
**dadurch gekennzeichnet, dass**
das Schlaf- oder Ruhemöbel einen elektromotorischen Möbelantrieb mit Verstellantrieben (7, 8) zum Verstellen von Möbelteilen und einer Steuereinrichtung (9) zur Ansteuerung der Verstellantrieben (7, 8) aufweist, wobei die Auswerteeinheit (9') mit der Steuereinrichtung (9) gekoppelt ist oder in die Steuereinrichtung (9) integriert ist, und dass die Matratze (30) mindestens eine in einem äußeren Bereich angeordnete Induktionsspule (18) aufweist, die mit dem Sensor (12) zur induktiven Stromversorgung des Sensors (12) verbunden ist.

2. Schlaf- oder Ruhemöbel nach Anspruch 1, bei dem die erfassten physiologischen Parameter eine Herzfrequenz, eine Atemfrequenz, ein Bewegungszustand und/oder ein Schnarchzustand der Person sind.

3. Schlaf- oder Ruhemöbel nach Anspruch 1 oder 2, bei dem die Auswerteeinheit (9') einen Filter, insbesondere einen Tief- oder Bandpassfilter zur Signalverarbeitung aufweist.

4. Schlaf- oder Ruhemöbel nach einem der Ansprüche 1 bis 3, bei dem die Auswerteeinheit (9') der Signale des mindestens einen Sensors (12) zusätzlich zur Erfassung und Auswertung von Vibrationen eingerichtet ist, die bei der Betätigung eines oder mehrerer der Verstellantriebe (7, 8) auftreten.

5. Schlaf- oder Ruhemöbel nach Anspruch 4, bei dem die Auswerteeinheit (9') dazu eingerichtet ist, eine Fehlfunktion und/oder eine Überlastung und/oder eine Nicht-Belastung eines oder mehrerer der Verstellantriebe (7, 8) im Betrieb zu ermitteln.

6. Schlaf- oder Ruhemöbel nach einem der Ansprüche 1 bis 5, bei der der Sensor (12) innerhalb der Matratze (30) zwischen zwei Schutzschichten (33) positioniert ist.

7. Schlaf- oder Ruhemöbel nach Anspruch 6, bei der die Schutzschichten (33) Moosgummi oder Filz aufweisen.

8. Schlaf- oder Ruhemöbel nach einem der Ansprüche 1 bis 7, aufweisend einen Federkern mit Federn (34), wobei mindestens eine der Federn (34) mit einem Ende auf dem Sensor (12) oder einer der diesen umgebenden Schutzschichten (33) aufliegt und wobei die mindestens eine der Federn (34) als Element zum Empfangen von Körperschall fungiert.

9. Schlaf- oder Ruhemöbel nach einem der Ansprüche 1 bis 8, bei dem die Matratze (30) einen von außen zugänglichen Steckkontakt (16) zur Kontaktierung des Sensors (12) aufweist.

10. Schlaf- oder Ruhemöbel nach einem der Ansprüche 1 bis 9, bei dem die Matratze (30) mindestens zwei leitende Kontaktflächen (17) zur Kontaktierung des Sensors (12) aufweist.

## Claims

1. Piece of sleeping or reclining furniture, in particular a bed (1), comprising:
- a mattress (30) having at least one piezoelectric or electromagnetic sensor (12) embedded in a foam material (32) of the mattress (30) for detecting vibrations, motion and/or sound, wherein the mattress (30) has at least one sensor plate (12'), which acts as an element for receiving structure-borne sound and to which the at least one sensor (12) is coupled,
- further comprising an evaluation unit (9'), which is connected to the sensor (12) and is designed to process and evaluate the signals from the at least one sensor (12) and to detect physiological parameters of a person using the piece of sleeping or reclining furniture,
**characterized in that**
the piece of sleeping or reclining furniture has an electromotive furniture drive comprising adjusting drives (7, 8) for adjusting furniture parts and comprising a control device (9) for controlling the adjusting drives (7, 8), wherein the evaluation unit (9') is coupled to the control device (9) or is integrated in the control device (9), and **in that** the mattress (30) has at least one induction coil (18), which is arranged in an outer region and is connected to the sensor (12) in order to inductively supply power to the sensor (12).

2. Piece of sleeping or reclining furniture according to claim 1, in which the detected physiological parameters are a heart rate, a respiratory rate, a motion state and/or a snoring state of the person.

3. Piece of sleeping or reclining furniture according to claim 1 or 2, in which the evaluation unit (9') has a filter, in particular a low-pass or band-pass filter for signal processing.

4. Piece of sleeping or reclining furniture according to any one of claims 1 to 3, in which the evaluation unit (9') for the signals from the at least one sensor (12) is additionally designed to detect and evaluate vibrations that occur on actuation of one or more of the adjusting drives (7, 8).

5. Piece of sleeping or reclining furniture according to claim 4, in which the evaluation unit (9') is designed to determine a malfunction and/or an overloading and/or a non-loading of one or more of the adjusting drives (7, 8) during operation.

6. Piece of sleeping or reclining furniture according to any one of claims 1 to 5, in which the sensor (12) is positioned within the mattress (30) between two protective layers (33).

7. Piece of sleeping or reclining furniture according to claim 6, in which the protective layers (33) comprise sponge rubber or felt.

8. Piece of sleeping or reclining furniture according to any one of claims 1 to 7, comprising a spring core having springs (34), wherein at least one of the springs (34) bears with one end on the sensor (12) or on one of the protective layers (33) surrounding the latter, and wherein the at least one of the springs (34) acts as an element for receiving structure-borne sound.

9. Piece of sleeping or reclining furniture according to any one of claims 1 to 8, in which the mattress (30) has a plug contact (16) for contacting the sensor (12), said plug contact being accessible from outside.

10. Piece of sleeping or reclining furniture according to any one of claims 1 to 9, in which the mattress (30) has at least two conductive contact areas (17) for contacting the sensor (12) .

## Revendications

1. Meuble de couchage ou de repos, en particulier un lit (1), présentant
- un matelas (30) avec au moins un capteur (12) à fonctionnement piézoélectrique ou électromagnétique intégré dans le matériau en mousse (32) du matelas (30) et servant à la détection des vibrations, du mouvement et/ou des bruits, le matelas (30) présentant au moins une plaque de capteur (12'), qui sert d'élément de réception du bruit structurel et avec laquelle ledit au moins un capteur (12) est relié,
- présentant en outre une unité d'évaluation (9') reliée au capteur (12) et conçue pour traiter et évaluer les signaux du ledit au moins un capteur (12) et pour saisir les paramètres physiologiques d'une personne utilisant le meuble de couchage ou de repos,
**caractérisé en ce que**
le meuble de couchage ou de repos présente un entraînement pour meuble à moteur électrique avec des entraînements de réglage (7, 8) servant au réglage des parties du meuble et d'un dispositif de commande (9) pour commander les entraînements de réglage (7, 8), l'unité d'évaluation (9') étant reliée au dispositif de commande (9) ou intégrée dans le dispositif de commande (9) et que
le matelas (30) présente au moins une bobine d'induction (18) disposée dans une partie extérieure, qui est reliée au capteur (12) pour l'alimentation électrique inductive du capteur (12).

2. Meuble de couchage ou de repos selon la revendication 1, dans lequel les paramètres physiologiques saisis sont une fréquence cardiaque, une fréquence respiratoire, un état cinématique et/ou un état de ronflement de la personne.

3. Meuble de couchage ou de repos selon la revendication 1 ou 2, dans lequel l'unité d'évaluation (9') présente un filtre, en particulier un filtre passe-bas ou filtre passe-bande, servant au traitement des signaux.

4. Meuble de couchage ou de repos selon l'une des revendications 1 à 3, dans lequel l'unité d'évaluation (9') des signaux dudit au moins un capteur (12) est conçue en plus pour saisir et évaluer les vibrations produites par l'actionnement d'un ou de plusieurs des entraînements de réglage (7, 8).

5. Meuble de couchage ou de repos selon la revendication 4, dans lequel l'unité d'évaluation (9') est conçue pour détecter un dysfonctionnement et/ou une surcharge et/ou une absence de charge d'un ou de plusieurs des entraînements de réglage (7, 8) pendant le fonctionnement.

6. Meuble de couchage ou de repos selon l'une des revendications 1 à 5, dans lequel le capteur (12) est positionné au sein du matelas (30) entre deux couches de protection (33).

7. Meuble de couchage ou de repos selon la revendication 6, dans lequel les couches de protection (33) présentent du caoutchouc mousse ou du feutre.

8. Meuble de couchage ou de repos selon l'une des revendications 1 à 7, présentant un noyau à ressorts avec des ressorts (34), une extrémité d'au moins un des ressorts (34) reposant sur le capteur (12) ou sur l'une de couches de protection (33) l'entourant et ledit au moins un des ressorts (34) servant d'élément de réception du bruit structurel.

9. Meuble de couchage ou de repos selon l'une des revendications 1 à 8, dans lequel le matelas (30) présente un contact enfichable (16) accessible d'extérieur servant à la mise en contact du capteur (12).

10. Meuble de couchage ou de repos selon l'une des revendications 1 à 9, dans lequel le matelas (30) présente au moins deux surfaces de contact conductrices (17) pour la mise en contact du capteur (12).
